(19) Europäisches Patentamt
European Patent Office
Office européen des brevets

(11) Veröffentlichungsnummer: **0 185 139**
**A1**

(12) # EUROPÄISCHE PATENTANMELDUNG

(21) Anmeldenummer: **85110623.7**

(22) Anmeldetag: **23.08.85**

(51) Int. Cl.[4]: **A 61 N 5/06**
H 02 P 7/00, G 05 F 1/10
G 01 J 1/42

(30) Priorität: **29.08.84 DE 3431629**
**10.07.85 DE 3524577**

(43) Veröffentlichungstag der Anmeldung:
**25.06.86 Patentblatt 86/26**

(84) Benannte Vertragsstaaten:
**AT BE CH DE FR GB IT LI LU NL SE**

(71) Anmelder: **AVARIS AG**
**Seilergraben 49**
**CH-8001 Zürich(CH)**

(72) Erfinder: **Kerschgens, Johann Josef**
**St.-Gotthard-Strasse 44**
**Altdorf(CH)**

(54) **UV-Bestrahlungsgerät.**

(57) Bestrahlungsgerät, kombiniert aus einem Gebläse (140) und einer Strahlungsquelle (33), die ultraviolette Strahlung (UV-Strahlung) abgibt und über einen Vorwiderstand (41) an die Netzspannung einer Wechselspannungsquelle angeschlossen ist, wobei die Strahlungsquelle mit dem vom Gebläse erzeugten Luftstrom zusammenwirkt und wobei die Drehzahl des Gebläses in Abhängigkeit von der Erhitzung der UV-Lampe geregelt wird. Die Regelung der Drehzahl des Gebläses erfolgt in Abhängigkeit von der emittierten UV-Strahlung der UV-Lampe.

EP 0 185 139 A1

./...

Croydon Printing Company Ltd

FIG.1

0185139



II/p/M 366

AVARIS AG, Seilergraben 49, Zürich (Schweiz)

Bestrahlungsgerät

Die vorliegende Erfindung betrifft ein Bestrahlungsgerät, kombiniert aus einem Gebläse und einer Strahlungsquelle, die ultraviolette Strahlung (UV-Strahlung) abgibt und über einen Vorwiderstand an die Netzspannung einer Wechselspannungsquelle angeschlossen ist, wobei die Strahlungsquelle mit dem vom Gebläse erzeugten Luftstrom zusammenwirkt und wobei die Drehzahl des Gebläses in Abhängigkeit von der Erhitzung der UV-Lampe geregelt wird.

Ein derartiges Bestrahlungsgerät ist bereits aus der DE-OS 33 22 071 bekannt. Dabei wird durch Regelung des Kühlluftstroms des Gebläses in Abhängigkeit von der Temperatur der UV-Lampe erreicht, daß die Temperatur der UV-Lampe auf einer ganz bestimmten Höhe gehalten werden kann, bei der diese eine vorgegebene UV-Strahlungsleistung und Wellenlänge abgibt, die für die gewünschten Zwecke die optimale Strahlung darstellt. Dabei erfolgt die Temperaturerfassung über NTC-Widerstände, die mit einem Motordrehzahleinstelltrimmer zur Arbeitspunkteinstellung (Temperatureinstellung) der UV-Lampe in Reihe geschaltet sind. Es hat sich nun herausgestellt, daß die Temperaturerfassung und Regelung der Drehzahl des Gebläses über NTC-Widerstände Nachteile hat, und zwar dahingehend, daß das Regelverhalten im Kaltzustand stark gegenüber dem betriebswarmen Zustand veränderlich ist, so daß es zu erheblichen Strahlungsleistungsdifferenzen kommt und eine derartige Regelung sehr träge ist.

Der Erfindung liegt nun die Aufgabe zugrunde, ausgehend von einem Bestrahlungsgerät der eingangs beschriebenen Art, dies derart zu verbessern, daß die Regelung des Kühlluftstromes des Gebläses in Abhängigkeit von der Temperatur der UV-Lampe praktisch unabhängig ist von dem Betriebszustand des Gerätes, wobei die Ansprechzeiten der Drehzahlregelung des Gebläses relativ kurz sind.

Erfindungsgemäß wird dadurch erreicht, daß die Regelung der Drehzahl des Gebläses in Abhängigkeit von der emittierten UV-Strahlung der UV-Lampe erfolgt. Dabei wird erfindungsgemäß zur Messung der Intensität der UV-Strahlung ein Foto-Element, insbesondere ein Fotowiderstand oder eine Fotozelle, verwendet, der ein UV-Filter vorgeschaltet ist, das ausschließlich für UV-Strahlung durchlässig ist. Die Erfindung geht dabei von der Erkenntnis aus, daß bei einer bestimmten Temperatur der UV-Lampe diese eine bestimmte UV-Strahlungsleistung abgibt, d.h. erhöht sich die Temperatur der UV-Lampe, so erhöht sich die UV-Strahlungsleistung,und erniedrigt sich die Temperatur der UV-Lampe, so nimmt die UV-Strahlungsleistung ab. Demnach ist die abgegebene UV-Strahlungsleistung proportional der Temperatur der UV-Lampe. Indem von dem erfindungsgemäß vorgesehenen Fotoelement nunmehr ausschließlich die UV-Strahlungsleistung erfaßt wird, kann somit die temperaturabhängige Regelung der Gebläsedrehzahl erfolgen, um einen bestimmten Arbeitspunkt der UV-Lampe konstant zu halten. Dabei zeichnet sich die Erfindung dadurch aus, daß sehr kurze Ansprechzeiten möglich sind und darüber hinaus das Meßverhalten des Fotoelementes unabhängig ist von der Betriebstemperatur des Bestrahlungsgerätes.

Erfindungsgemäß ist vorgesehen, daß das Fotoelement mit dem vorgeschalteten nur UV-Strahlung durchlassenden Filter hinter dem Reflektor der UV-Lampe eingebaut ist, wobei in dem Reflektor hierzu eine Öffnung vorgesehen ist und der Filter unmittelbar hinter oder in der Öffnung eingebaut werden kann.

Weiterhin kann in zuverlässiger Weise und mit einfachen schaltungstechnischen Maßnahmen eine temperaturabhängige Regelung der Strahlungsintensität eines UV-Strahlers durchgeführt werden, indem eine Gasentladungslampe von dem UV-Strahler bestrahlt wird und die Durchbruchspannung der Gasentladungslampe als Regelgröße für die Regelung der Strahlertemperatur des UV-Strahlers verwendet wird. Die Erfindung basiert dabei auf der Erkenntnis, daß die für eine bestimmte Gasentladungslampe genau definierte Durchbruchspannung vom Abstand der Elektroden, von der Beschaffenheit der Gasfüllung und dem Ionisierungsgrad des Gases im wesentlichen abhängig ist. Bei vorgegebenem Elektrodenabstand und vorgegebener Gasfüllung hängt somit die Durchbruchspannung vom Ionisierungsgrad des Gases ab. Die Erfindung macht sich dabei zunutze, daß durch das Zuführen energiereicher Strahlung der Ionisierungsgrad von Gasen beeinflußt werden kann. Indem erfindungsgemäß in dem Abstrahlungsbereich der zu regelnden UV-Lampe die Gasentladungslampe angeordnet ist, wird demnach deren Durchbruchspannung von der UV-Strahlung des UV-Strahlers beeinflußt, und zwar derart, daß bei Anstieg der Strahlungsintensität die Durchbruchspannung der Gasentladungslampe sinkt. Demnach ist die Veränderung der Durchbruchspannung unmittelbar abhängig von der Veränderung der Strahlungsintensität des UV-Strahlers und kann somit als Regelgröße verwendet werden. Erfindungsgemäß kann es vorteilhaft sein, wenn als Gasentladungslampe eine Glimmlampe verwendet wird. Hierdurch ergibt sich eine besonders preisgünstige Durchführung des erfindungsgemäßen Verfahrens.

Durch das erfindungsgemäße Verfahren wird demnach ein elektrischer Regelwert erzeugt, der direkt der zugeführten Strahlungsenergie proportional ist. Da sichtbares Licht oder Infrarotlicht relativ energiearme Strahlungen sind, werden sie bei der Messung entsprechend schwach bewertet, und umgekehrt wird UV-B oder UV-C-Strahlung entsprechend stärker bewertet. Erfindungsgemäß ist es demnach nicht erforderlich, Filtergläser zu verwenden oder eine Temperaturkompension vorzunehmen, solange keine frequenzselektive Messung durchgeführt werden soll. Es liegt jedoch ebenfalls im Rahmen der Erfindung, auch frequenzselektiv zu messen, d.h. in den Strahlengang zwischen UV-Lampe und Gasdrucklampe einen Filter vorzusehen, so daß eine Regelung in Abhängigkeit von einer bestimmten Wellenlänge durchgeführt werden kann.

Die Zeichnungen veranschaulichen die Erfindung an mehreren Ausführungsbeispielen, und zwar zeigt

Fig. 1 einen Querschnitt durch das erfindungsgemäße Bestrahlungsgerät, teilweise geschnitten,

Fig. 2 eine Draufsicht auf das Gerät gemäß Fig. 1

Fig. 3 verschiedene Spektralverteilungen nach Reflexion der UV-Lampe an unterschiedlichen Reflektoren,

Fig. 4 eine erfindungsgemäße elektrische Schaltung einer UV-Lampe,

Fig. 5 ein Schaltbild eines Netzteils für ein Gebläse,

Fig. 6 eine Anzeigeschaltung für die Betriebstemperatur der UV-Lampe sowie für die Kühlleistung des Gebläses,

Fig. 7 ein erfindungsgemäßes Gesamtschaltbild für den Betrieb zweier UV-Lampen mit geregelter Kühlung,

Fig. 8 eine Seitenansicht eines erfindungsgemäßen Föns mit UV-Aufsatz und Abstandhalter,

Fig. 9 eine Draufsicht auf den Abstandhalter aus der Sicht des zu bestrahlenden Objektes,

Fig. 10 eine perspektivische Ansicht eines erfindungsgemäßen Fönes mit abgenommenem UV-Aufsatz,

Fig. 11 eine Seitenansicht des Fönes gemäß Fig. 10 in geöffnetem Zustand,

Fig. 12 eine erfindungsgemäße Verbindungsvorrichtung zwischen UV-Aufsatz und Fön,

Fig. 13 eine Draufsicht auf die Frontseite des UV-Aufsatzes,

Fig. 14 eine Schnittansicht durch den Frontbereich des erfindungsgemäßen UV-Aufsatzes gemäß Fig. 13 entlang der Linie 15-15.

Fig. 15 einen Schnitt durch den Strahlervorsatz gemäß Fig. 13 und

Fig. 16 ein Prinzipschaltbild einer erfindungsgemäßen UV-Meßschaltung.

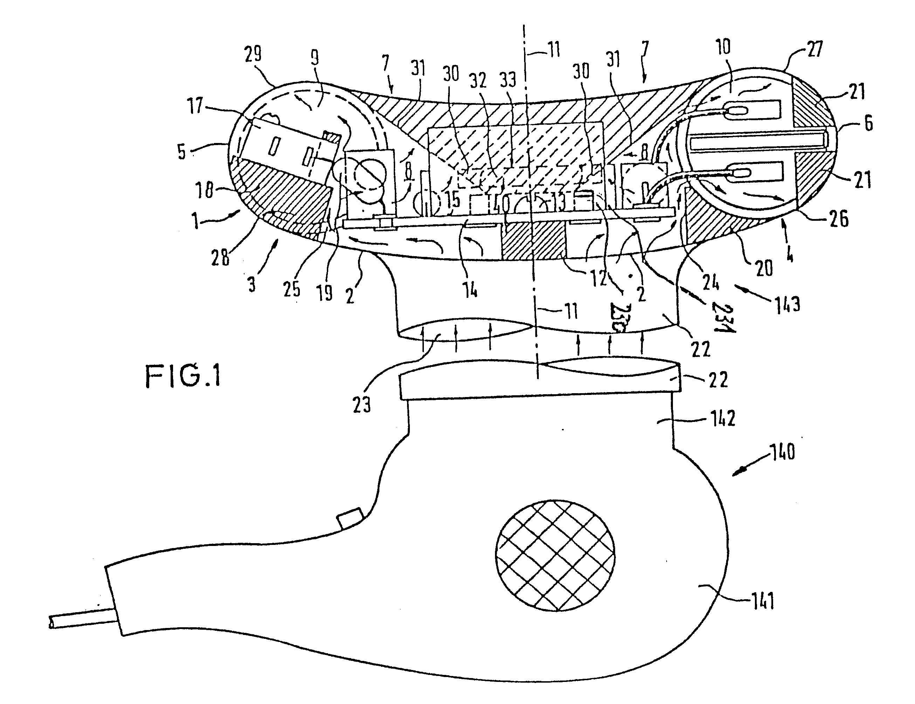

Das Gehäuse 1 des in Fig. 1 abgebildeten erfindungsgemäßen Bestrahlungsgerätes weist eine von den Bestrahlungsseite her betrachtet - das ist die in Fig. 1 oben liegende Seite - konkave Rückwand 2 auf, die an ihren äußeren Enden 3 und 4 in eine teilkreisförmige Abrundung 5 und 6 übergeht. Die Abrundungen 5 und 6 grenzen von der Bestrahlungsseite her gesehen an einen Reflektorraum 7. Im Innern 8 des Gehäuses 1 sind jeweils endseitig, angepaßt an die Abrundung 5 bzw. 6, Kondensatoren 9 bzw. 10 angeordnet. Mittig zur Längsachse 11 des Gehäuses 1 ist eine mit diesem verbundene Leiterplattenbefestigung 12 vorgesehen, an der mittels einer Befestigungsschraube 13 eine Leiterplatte 14 gehalten wird. Die Längsachse 11 steht senkrecht auf der Oberfläche 15 der Leiterplatte 14. Auf der Leiterplatte 14 sind verschiedene Bauteile für eine im Reflektorraum 7 angeordnete UV-Lampe 33 angeordnet. Auf der in Fig. 1 linken Seite, ist vor dem Kondensator 9 ein Mikroschalter 17 auf einer zum Gehäuse 1 gehörigen Schalterbefestigung 18 angebracht, mit dem das Bestrahlungsgerät ein- und ausgeschaltet werden kann. Das Ein- und Ausschalten erfolgt durch Druck auf einen von außen zugänglichen Druckknopf des Mikroschalters 17 (nicht dargestellt). Ein an den Mikroschalter 17 angeschlossener Thermoschalter 19 überwacht die Temperatur im Innern 8 des Gehäuses 1 und schaltet beim Überschreiten einer vorbestimmten Temperatur - z.B. 80° - das Bestrahlungsgerät aus. Jeder Kondensator 9, 10 wird von einer Halterung 20 abgestützt und von einem Verdrehschutz 21 gesichert (in der Fig. 1 nur bei dem Kondensator 10 dargestellt). Vorzugsweise ist die Halterung 20 federnd ausgebildet.

An die Rückwand 2 schließt sich koaxial zur Längsachse 11 eine Aufstecktülle 22 an, die zum Aufsetzen des Gehäuses 1 auf ein Gebläse 140 dient.

Nach einer Weiterbildung der Erfindung ist vorgesehen, daß das Gebläse 140 ein Fön 141 ist, auf dessen Luftaustrittsstutzen 142 die Aufstecktülle 22 durch Klemmsitz befestigt werden kann. Für die Befestigung kann nach einer anderen Ausbildung auch ein grobes Schraubgewinde oder es können radial angreifende Feststellschrauben vorgesehen sein.

Die von dem Gebläse 140 abgegebene Luftströmung ist in Fig. 1 durch Pfeile angedeutet, die zunächst die Öffnung 23 der Aufstecktülle 22 durchsetzt und in ihrem weiteren Verlauf durch die zwischen Leiterplatte 14 und Kondensator 9 bzw. 10 gebildeten Lüftführungskanäle 24 bzw. 25 strömt. Hieran anschließend überstreicht ein Teil des Luftstromes die Kondensatoren 9 und 10 und tritt dann aus dem Gehäuse 1 durch im Bereich der Kondensatoren 9 bzw. 10 vorgesehene Auslässe 26,27,28 und 29 aus. Dieser Teilluftstrom umspült somit die Kondensatoren 9 und 10. Ein weiterer Teilluftstrom überstreicht die auf der Leiterplatte 14 angeordnete UV-Lampenfassung 30 und findet dann über andere, nicht dargestellte Luftauslässe seinen Weg aus dem Gehäuse 1 heraus.

Innerhalb des Gehäuses 1 ist hinter dem Reflektorraum 7 ein Fotoelement 230 angeordnet, dem ein Filter 231 vorgelagert ist, der nur für UV-Strahlung durchlässig ist. Hierfür ist im Reflektor eine Öffnung vorgesehen, was nicht dargestellt ist, und der Filter 231 kann in oder unmittelbar hinter der Öffnung angeordnet sein.

Es ist vorgesehen, daß die den Reflektorraum 7 begrenzenden Wände 31 Öffnungen aufweisen, durch die ein Teil des Luftstromes strömt und dadurch den Glaskolben 32 der UV-Lampe 33 sowie den Reflektorraum 7 kühlt.

Es kann auch vorgesehen sein, daß im oberen Teil des Gehäuses 1 zwei Ausschnitte vorgesehen sind (nicht dargestellt), die von oben (Fig. 1) ein Einlöten der Lampenanschlußdrähte der UV-Lampe 33 in die Leiterplatte 14 ermöglichen. Die Ausschnitte werden durch das spätere Aufsetzen von Abdeckungen (nicht dargestellt) für die Lampenbefestigung durch Endkappen verdeckt. Lampenhalterung und deren Abdeckungen sind dann so ausgebildet, daß möglichst geringe Öffnungen zum Innern 8 des Gehäuses 1 verbleiben, um eine Erwärmung des Innern 8 durch die UV-Lampe 33 zu vermeiden.

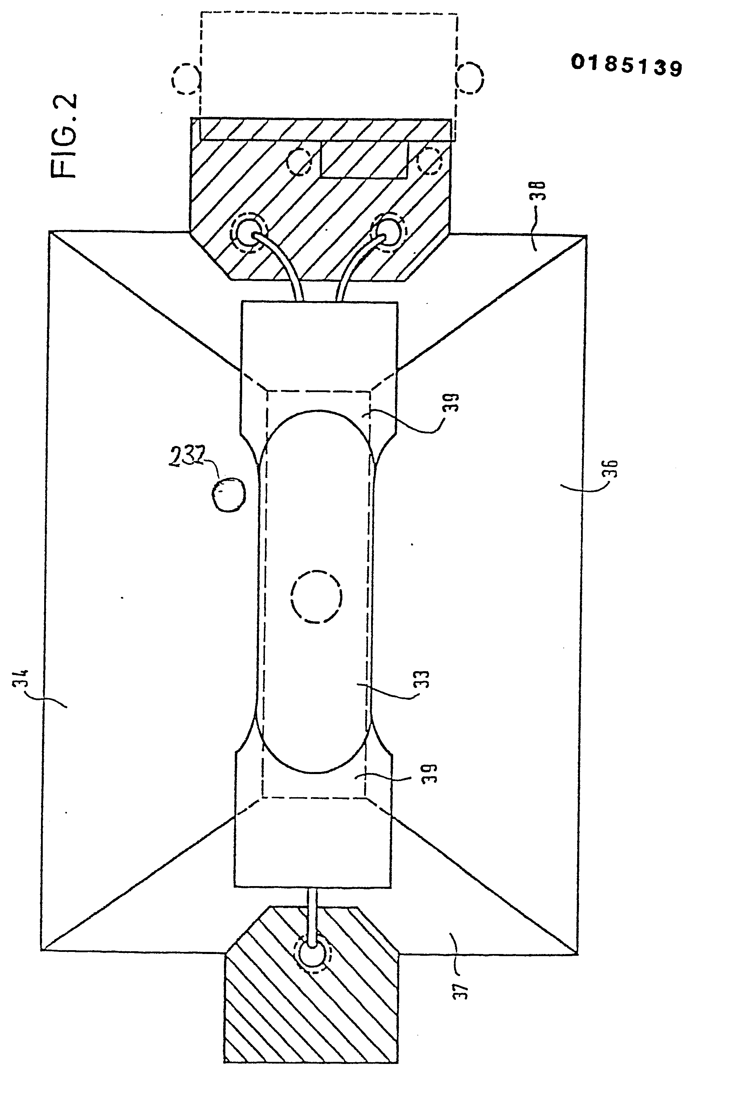

In der Fig. 2 ist eine Draufsicht auf die UV-Lampe 33 sowie deren im Reflektorraum 7 angeordneter Reflektor 34 aus der Richtung des zu bestrahlenden Objektes dargestellt. Der Reflektor 34 besteht vorzugsweise aus fünf ebenen Reflektorflächen 35 bis 39, die so angeordnet sind, daß eine optimale Reflektion der von der UV-Lampe 33 ausgehenden Strahlung erfolgt. Dabei ist eine Öffnung 232 im Reflektor zu erkennen, hinter der das für UV-Strahlung ausschließlich durch-

lässige Filter angeordnet ist, das das Fotoelement abdeckt.

Es ist vorgesehen, daß die Reflektorflächen 35 bis 39 mit Öffnungen versehen sind, durch die ein Teil des von dem Gebläse erzeugten Luftstromes zur Kühlung der UV-Lampe tritt.

Nach einer weiteren Ausführung der Erfindung können auch mehrere UV-Lampen in dem Reflektorraum 7 angeordnet sein.

Insbesondere aus der Fig. 2 ist es ersichtlich, daß die gesamte Reflektorfläche des Reflektors 34 zur Reflektion der UV-Strahlung zur Verfügung steht. Dieses ist bei bekannten Bestrahlungsgeräten nicht der Fall, denn innerhalb des Reflektorraumes ist dort noch der als Vorwiderstand wirkende IR-Strahler angeordnet. Hierdurch wird die effektive Reflektorfläche verkleinert, so daß die abgestrahlte Strahlungsleistung der UV-Lampe bei den bekannten Geräten kleiner als bei dem vergleichbaren erfindungsgemäßen Gerät ist.

Durch die Ausbildung des Reflektors 34 mit seinen fünf Reflektorflächen 35 bis 39 wird durch die erfindungsgemäße Vorrichtung das Bestrahlungsobjekt aus mehreren Richtungen mit divergierendem Licht bestrahlt, so daß auch nicht ebene Teile oder Flächen des Bestrahlungsobjektes, z. B. die Gesichtsfläche einer zu bestrahlenden Person, gleichmäßig bestrahlt werden. Unabhängig von dem unterschiedlichen Abstand zum Gerät erhält jede Stelle des Körpers eine nahezu gleiche Strahlungsdosis. Darüber hinaus ist der erfindungsgemäße Reflektor mit seinen ebenen Flächen gegenüber dem bei bekannten Vorrichtungen eingesetzten parabolförmigen Reflektor kostengünstiger und einfacher herzustellen.

Nach einer Weiterbildung der Erfindung ist vorgesehen, daß zur Unterdrückung bestimmter Spektralbereiche der UV-Bestrahlung und/oder langwelliger sichtbarer Strahlung und/oder IR-Strahlung zusätzlich im Reflektorraum 7 angeordnete Filtervorrichtungen vorhanden sind. Da die Spektralverteilung der auf den zu bestrahlenden Körper auftreffenden Strahlung auch im hohen Maße vom Material der Oberflächenschicht des Reflektors beeinflußt wird, wird dafür vorzugsweise ein Material verwendet, daß UV-A-Strahlung gut reflektiert. Derartige Materialien sind z. B. gelb eloxiertes Aluminium, hochglanzpolierter Edelstahl mit hohem Chrom-Anteil oder Chrom.

Um sich eine Vorstellung über die Abmessungen des beschriebenen Ausführungsbeispiels machen zu können, sollen einige Maße angegeben werden. Die Radien der Abrundungen 5 und 6 betragen etwa 140 mm; der Abstand zwischen der Unterseite der Leiterplatte 14 und der Gehäuserückwand 2 beträgt im Bereich der Längsachse 11 etwa 6 mm. Der Abstand zwischen der Oberfläche 15 der Leiterplatine 14 und dem Reflektor 34 sollte zweckmäßigerweise wegen der Befestigungsschraube 13 und aus verdrahtungstechnischen Gründen einen in der Fig. 1 mit 40 bezeichneten Abstand von etwa 3 mm aufweisen. Das erfindungsgemäße Bestrahlungsgerät wiegt insgesamt nur etwa 250 - 300 g, ist kaum größer als eine Zigarettenschachtel und kann deshalb leicht mitgeführt werden.

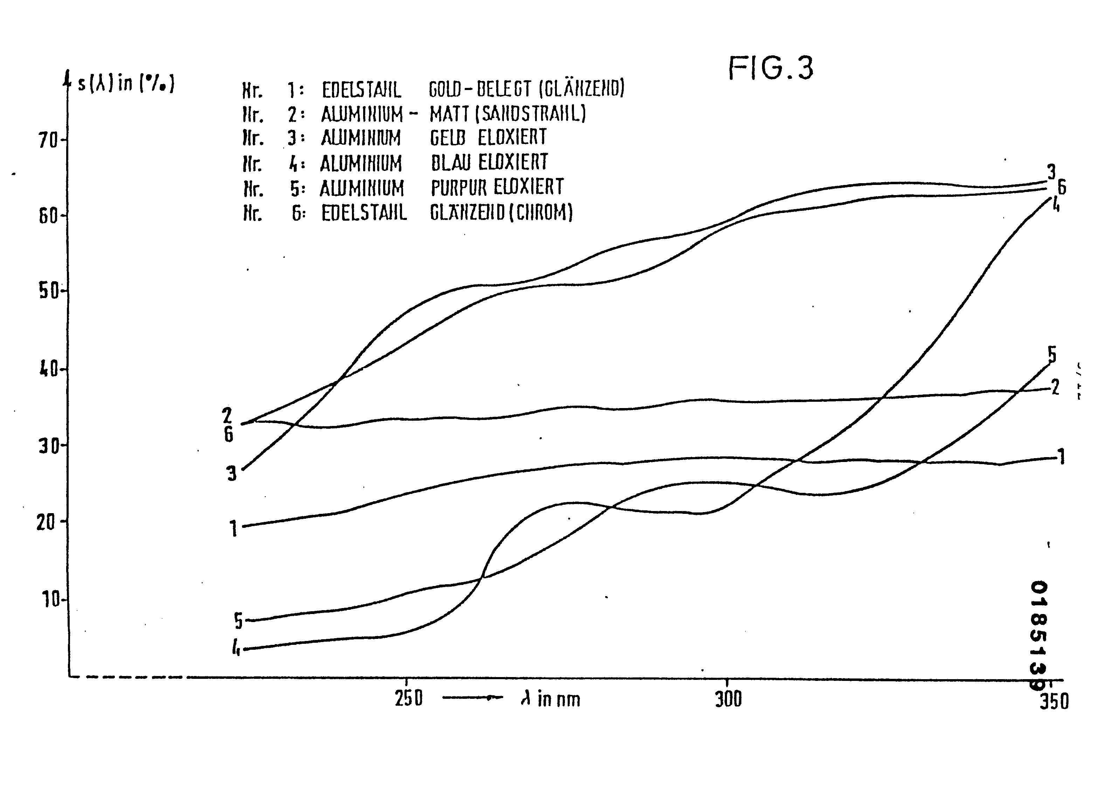

Die Fig. 3 zeigt die Spektralverteilung der in Richtung auf den zu bestrahlenden Körper emittierten UV-Strahlung für verschiedene Oberflächenschichten des Reflektors 34, wobei die Oberflächenschichten 3 (Aluminium gelb eloxiert) und 6 (Edelstahl glänzend (Chrom)) besonders bevorzugt sind.

Für die Zuführung von elektrischer Energie zu der UV-Lampe 33 ist am Fön 141 eine Buchse angeordnet, in die ein an der Aufstecktülle 22 angeordnetes Steckerteil beim Aufsetzen des Gehäuses 1 auf den Fön 141 eingreift.

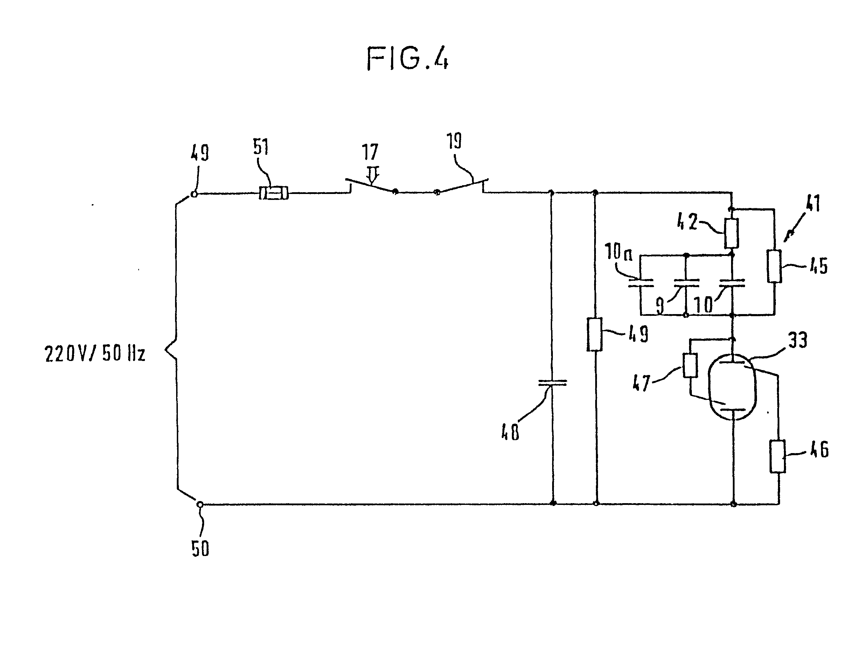

Die Fig. 4 zeigt die elektrische Schaltung der UV-Lampe 33. In Reihe zu der UV-Lampe 33 liegt ein aus ohm'schen und kapazitiven Widerständen zusammengesetzter Vorwiderstand 41. Der Vorwiderstand 41 besteht aus einem Leistungswiderstand 42, zu dem in Reihe drei parallel geschaltete Kondensatoren 9, 10 und 10a liegen. Parallel zu der aus dem Widerstand 42 und den Kondensatoren 9, 10 und 10a gebildeten Reihenschaltung liegt ein Widerstand 45. Bei Verwendung einer Quecksilberdampf-Hochdrucklampe OS 124 (Nennleistungsaufnahme 125 W, Brennerspannung 85 V $\pm$ 11 V bei einem Nennstrom von ca. 2A; Hersteller Osram GmbH) als UV-Lampe 33 wird ein kapazitiver Vorwiderstand bei Netzbetrieb (220 V / 50 Hz) von 30 µF benötigt. Somit besitzen die Kondensatoren 9, 10 und 10a jeweils eine Kapazität von 10 µF. Der Widerstand 42 hat einen Wert von 0,5 Ohm und dient zur Spitzenstrombegrenzung. Der Widerstand 45 dient zum Entladen der Kapazitäten nach Ausschaltung des Gerätes und hat einen Wert von etwa 100 k$\Omega$ . Gezündet wird die vorliegende UV-Lampe über die Widerstände 46 und 47.

Parallel zu der Reihenschaltung aus Vorwiderstand 41 und UV-Lampe 33 liegen eine Kapazität 48 sowie ein Widerstand 49. Diese Bauelemente dienen der Entstörung. Die Zuführung der Versorgungsspannung über die Klemmen 49 und 50 erfolgt über die Reihenschaltung einer Sicherung (Strombegrenzung) 51, dem Mikroschalter 17 und dem Thermoschalter 19.

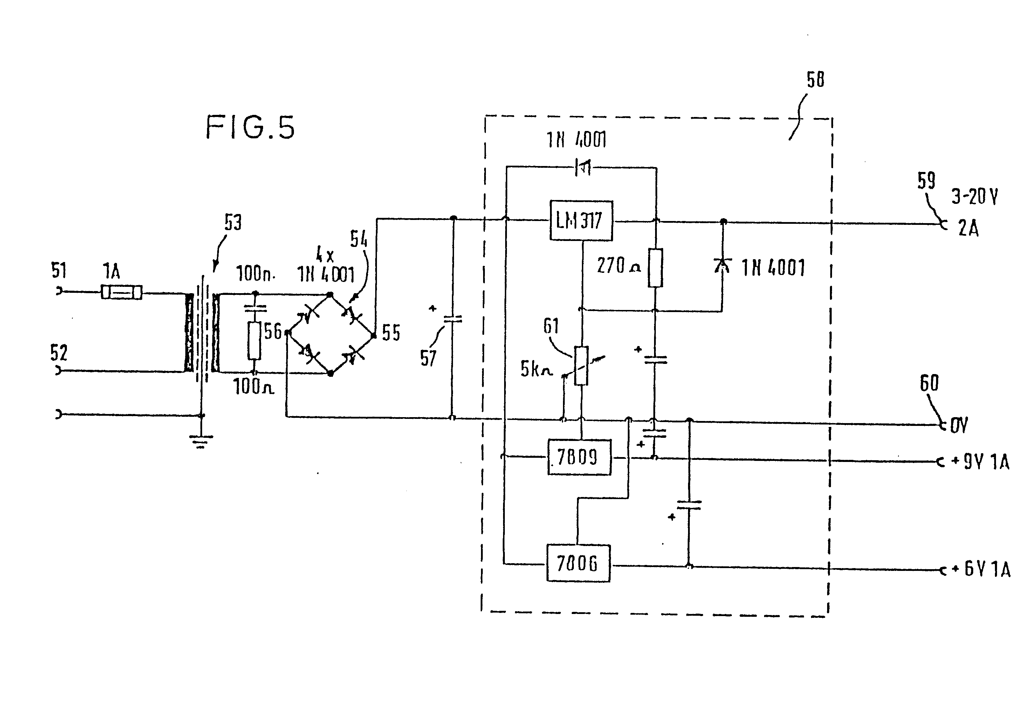

In der Fig. 5 ist ein Netzgerät dargestellt, das zur Versorgung des Antriebsmotors (Gleichstrommotor) des Fönes 141 dient. An die Eingangsklemmen 51 und 52 wird Netzspannung (220V / 50 Hz) gelegt, die mittels des Transformators 53 heruntergespannt und dem Brückengleichrichter 54 zugeführt wird. An den Ausgängen 55 und 56 des Brückengleichrichters 54 liegt dann eine Gleichspannung an, die von dem Kondensator 57 geglättet und einer Regel- und Stabilisierungsschaltung 58 zugeführt wird. die an den Ausgangsklemmen 59 und 60 anliegende Ausgangsspannung läßt sich mit Hilfe des Potentiometers 61 regeln. Da an die Ausgangsklemmen 59 und 60 der Gleichstrommotor des Fönes angeschlossen wird, ist dessen Drehzahl durch Verstellung des Potentiometers 61 einstellbar, wodurch die aus dem Fön 141 austretende Luftmenge gesteuert werden kann. Mit Änderung der Betriebstemperatur der UV-Lampe 33 variiert sowohl ihre Leistung als auch die Wellenlänge der abgegebenen Strahlung. An dem Potentiometer 61 läßt sich - wie schon beschrieben - die Luftmenge und damit die Kühlwirkung der UV-Lampe 33 steuern. Hiermit ist also die Möglichkeit geschaffen, sowohl auf die Leistung als auch auf die Wellenlänge der UV-Lampe 33 Einfluß zu nehmen. Je nach dem vorliegenden Bedürfnis kann das erfindungsgemäße Bestrahlungsgerät damit optimal eingestellt werden.

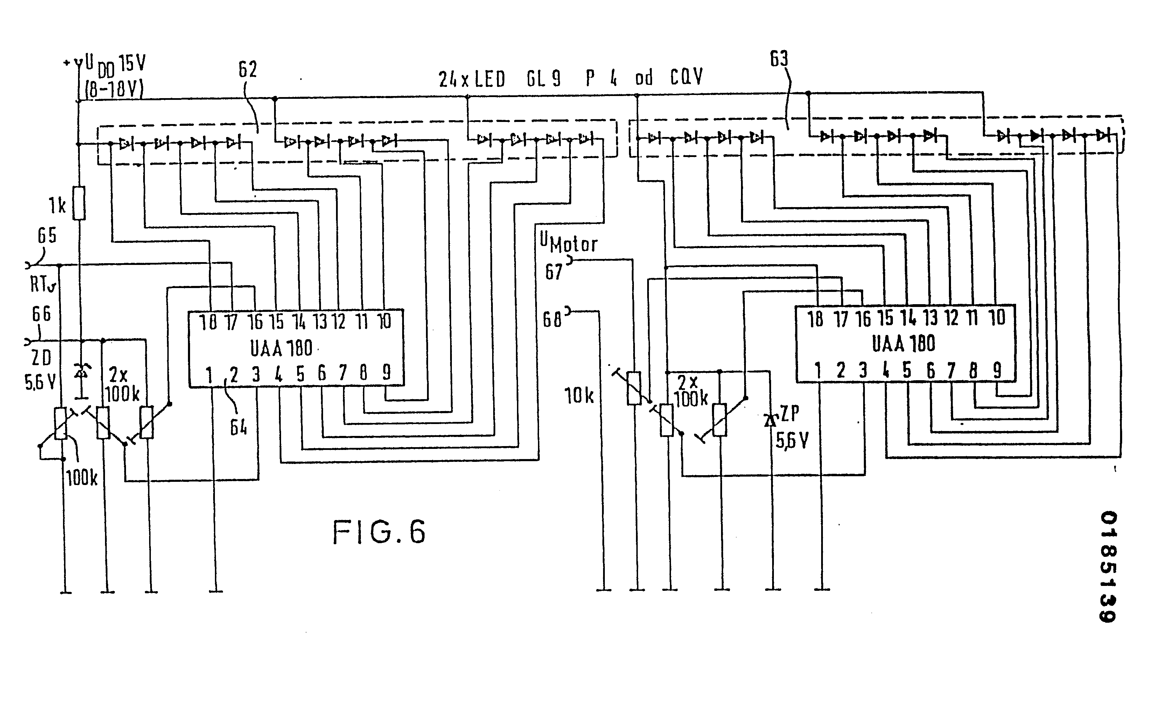

Die Fig. 6 zeigt eine Anzeigeschaltung, die zwei Leuchtdioden-Reihen 62 und 63 aufweist. Die Leuchtdiodenreihe 62 wird durch den integrierten Schaltkreis 64 (UAA 180) angesteuert und dient der Anzeige der in dem erfindungsgemäßen Bestrahlungsgerät vorherrschenden Temperatur. An die Klemmen 65 und 66 wird hierzu eine der Temperatur im Innern 8 des Gehäuses 1 proportionale Spannung angelegt, die in dem integrierten Schaltkreis 64 ausgewertet wird und eine entsprechende Anzahl von Leuchtdioden der Leuchtdioden-Reihe 62 zum Ansprechen bringt. Als Temperaturfühler kann beispielsweise ein NTC-Widerstand verwendet werden. Der NTC-Widerstand kann direkt oder indirekt mit dem Glaskolben der UV-Lampe 33 in Kontakt stehen, so daß die Anzahl der aufleuchtenden Leuchtdioden ein Maß für die Kolbentemperatur und somit für die abgegebene Strahlungsleistung und für die Wellenlänge der von der UV-Lampe 33 abgegebenen Strahlung ist. Die Anzeigeschaltung kann dabei so ausgeführt sein, daß sich bei steigender Temperatur die Anzahl der leuchtenden Leuchtdioden erhöht.

Die andere Leuchtdioden-Reihe 63 arbeitet in analoger Weise; ihr wird jedoch über die Klemmen 67 und 68 die Motorspannung des Föns 141 zugeführt, so daß die Anzahl der leuchtenden Leuchtdioden der Leuchtdioden-Reihe 63 ein Maß für die abgegebene Kühlluftmenge ist. Der Benutzer des Gerätes kann somit über die beiden Leuchtdiodenanzeigen einen gewünschten Arbeitspunkt der erfindungsgemäßen Vorrichtung einstellen.

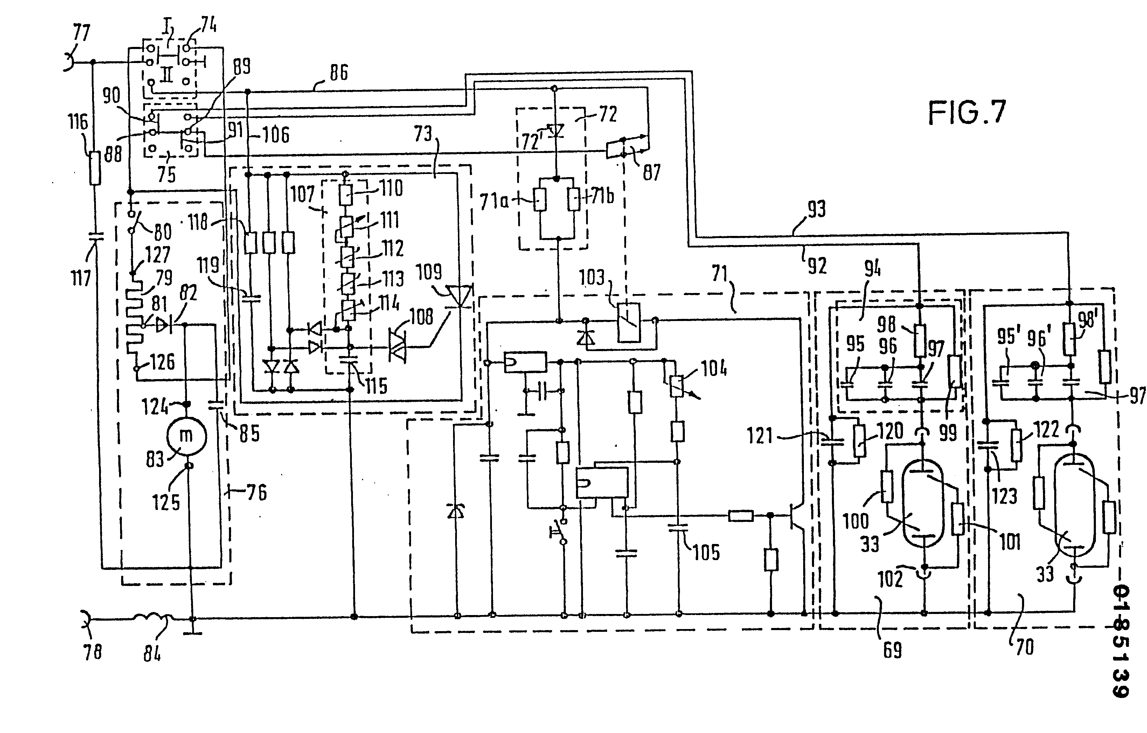

Die Fig. 7 zeigt ein Gesamtschaltbild für ein erfindungsgemäßes Bestrahlungsgerät, wobei zwei UV-Lampen und eine automatische Kühlluftregelung vorgesehen sind. Die Gesamtschaltung setzt sich aus zwei UV-Lampenschaltungen 69 und 70, einer Zeitschaltung 71, einer Versorgungsschaltung 72 für die Zeitschaltung 71, einer Phasenanschnittschaltung 73, einem Fönschalter 74, einem UV-Lampenschalter 75 und einer Fönschaltung 76 zusammen.

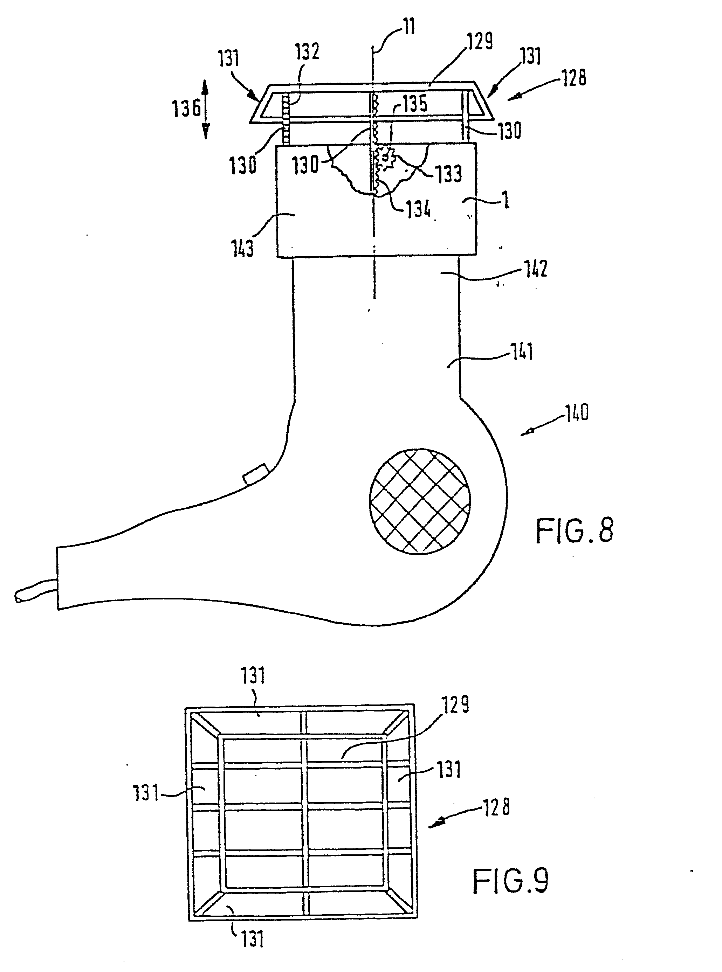

Wird an die beiden Klemmen 77 und 78 Netzspannung angelegt (220 V / 50 Hz) und befindet sich der Fönschalter 74 in der in Fig. 8 eingezeichneten Stellung I, so wird die Heizung 79 des Föns in Betrieb gesetzt. Die Heizung 79 besteht aus Widerstanddraht, der sich beim Stromdurchfluß erwärmt. In Reihe zur Heizung 79 liegt ein Temperaturschalter 80, der beim Überschreiten einer vorgegebenen Temperatur die Heizung 79 ausschaltet. Die Heizwicklung der Heizung 79 besitzt einen Abgriff 81, an dem eine Diode 82 angeschlossen ist. Die Diode ist mit dem einen Pol 124 eines Gleichstrommotors 83 verbunden; dessen anderer Pol 125 über die Drossel 84 mit der Klemme 78 in Verbindung steht. Bei dem Gleichstrommotor 83 (z.B. ein 12 V-Motor) handelt es sich um den Gebläsemotor des Föns. Parallel zu dem Gleichstrommotor 83 liegt ein Kondensator 85, der die Motorspannung glättet. In diesem Betriebszustand (Fönschalter 74 in Stellung I) ist der Fön in herkömmlicher Art und Weise zu benutzen.

Wird der Fönschalter 74 in seine Stellung II gebracht, so wird über die Leitung 86 bei geschlossenem Relaiskontakt 87, die Anschlüsse 88 und 89 des UV-Lampenschalter 75 an Spannung gelegt. Je nach Stellung der Schaltkontakte 90 und 91 können die UV-Lampenschaltungen 69 und 70 über die Leitungen 92 und 93 ein- bzw. ausgeschaltet werden. Die UV-Lampenschaltung 69 besteht aus einem Vorwiderstand 94, der drei parallel geschaltete Kondensatoren 95 bis 97 (jeweils 10 µF), einem dazu in Reihe liegenden Leistungswiderstand 98 (0,5 Ω) und einen parallel zu der Reihenschaltung von Leistungswiderstand 98 und Kondensator 97 liegenden Widerstand 99 (100 kΩ) umfaßt. In Reihe zu dem Vorwiderstand 94 liegt die UV-Lampe 33, deren Zündelektroden jeweils mit Widerständen 100 und 101 verbunden sind. Die Widerstände 100 und 101 besitzen einen Wert von 15 kΩ und dienen der Erzeugung der Zündspannung. Der in Fig. 7 untere Anschluß 102 ist über die Drossel 84 mit der Klemme 78 verbunden. Die drei Kondensatoren 95, 96 und 97 dienen somit als Vorwiderstand für die UV-Lampe 33. Der Widerstand 98 begrenzt den Spitzenstrom, und der Widerstand 99 dient der Kondensatorentladung nach dem Abschalten der UV-Lampenschaltung 69. Die UV-Lampenschaltung 70 ist ebenso wie die UV-Lampenschaltung 69 aufgebaut.

Die beiden UV-Lampenschaltungen 69 und 70 können nur in Betrieb gesetzt werden, wenn der Relaiskontakt 87 geschlossen ist. Dieser Relaiskontakt gehört zu einem Relais 103 der Zeitschaltung 71. Zeitbestimmende Glieder der Zeitschaltung 71 sind das Potentiometer 104 und der Kondensator 105. An dem Potentiometer 104 kann die Anzugszeit des Relais 103 eingestellt werden. Hiermit läßt sich also die Einschaltdauer der UV-Lampenschaltungen 69 und 70

bestimmen. Da Zeitschaltungen aus dem Stand der Technik bekannt sind, soll auf die nähere Ausführung der Zeitschaltung 71 nicht eingegangen werden. Die Versorgungsschaltung 72 für die Zeitschaltung 71 besteht aus einer Diode 72' und zwei parallel geschalteten Leistungswiderständen 71a,b. Diese sind so angeordnet, daß sie durch den Luftstrom des Fönes gekühlt werden, so daß die erzeugte Wärme abgeführt werden kann.

Befindet sich der Fönschalter 74 in seiner Stellung II, so wird über die Leitung 106 die Phasenanschnittschaltung 73 mit Spannung versorgt. Die Phasenanschnittschaltung 73 weist ein anschnittswinkelbestimmendes Glied 107 auf, daß ein Diac 108 ansteuert, welches mit dem Gate eines Tyristors 109 in Verbindung steht. Der Tyristor 109 liegt in der beschriebenen Betriebsstellung des Fönschalters 74 in Reihe mit der Fönschaltung 76, wobei die Reihenschaltung von Tyristor 109 und Fönschaltung 76 über die Drossel 84 an Betriebsspannung (220 V / 50 Hz) liegt. Mit Variation des Phasenanschnittswinkels der Phasenanschnittschaltung 73 läßt sich die Versorgungsspannung des Fönes variieren, wodurch die Motordrehzahl des Gleichstrommotors 83 veränderbar ist. Die Versorgungsspannung liegt in dieser Betriebsart an der aus einem Teilstück der Heizung 79 (vom Anschluß 120 der Heizwicklung bis zum Abgriff 81), der Diode 82 und dem Motor 83 gebildeten Reihenschaltung an.

Das den phasenanschnittwinkelbestimmende Glied 107 der Phasenanschnittsschaltung 73 wird durch die Reihenschaltung von Widerstand 110,Potentiometer 111, Fotoelement 112, Fotoelement 113, Trimmer 114 und Kondensator 115 gebildet. Die beiden Fotoelemente 112 und 113 sind jeweils einer der UV-Lampenschaltungen 69 bzw. 70 zugeordnet und sind vorzugsweise Fotowiderstände. Jedes der beiden Fotoelemente befindet sich in unmittelbarer Nähe der entsprechenden UV-Lampe, so daß die von der jeweiligen UV-Lampe ausgehende UV-Strahlung von dem entsprechenden Fotoelement registriert wird. Je nach Strahlungsbeaufschlagung der Fotoelemente verändern diese ihren Widerstandswert (Fotowiderstand), wodurch der Phasenanschnittswinkel der Phasenanschnittsschaltung 73 verändert und damit auf die Motordrehzahl des Gleichstrommotors 83 Einfluß genommen wird. Mit Veränderung der Motordrehzahl ändert sich die von dem Fön abgegebene Kühlluftmenge, die bei der erfindungsgemäßen Vorrichtung zur Kühlung der UV-Lampe 33 verwendet wird. Im nachfolgenden wird nur von einer UV-Lampe gesprochen, es können natürlich auch mehrere - so wie in der Fig. 7 z. B. zwei - UV-Lampen verwendet werden. Zur Beschreibung des Regelkreises soll davon ausgegangen werden, daß die Temperatur der UV-Lampe 33 der UV-Lampenschaltung 69 steigt. Hierdurch verändert auch der in unmittelbarer Nachbarschaft liegende Foto-Widerstand 112 seinen Widerstandswert, wodurch sich der Phasenanschnittswinkel der Phasenanschnittsschaltung 73 derart ändert, daß der Fönschaltung 76 eine höhere Spannung zugeführt wird. Damit erhöht sich auch die Drehzahl des Gleichstrommotors 83, wodurch eine größere Luftmenge aus dem Fön 141 ausgestoßen wird, die wiederum zur intensiveren Kühlung der UV-Lampe 33 führt, so daß eine bestimmte Temperatur an der UV-Lampe 33 erreicht wird.

Erniedrigt sich nun die Temperatur an der UV-Lampe 33, so verringert sich die von ihr erzeugte UV-Strahlung, so daß nun die Regelschaltung im entgegengesetzten analogen Sinne wirkt. Der Foto-Widerstand 113 ist der UV-Lampenschaltung 70 zugeordnet.

Der Arbeitspunkt der Strahler kann an dem Trimmer 114 der Motorregelung genau justiert werden. Mit Hilfe des Potentiometers 111 läßt sich manuell auf die Regelschaltung Einfluß nehmen.

Da in der in Fig. 7 dargestellten erfindungsgemäßen Schaltung an verschiedenen Stellen starke Störspannungen auftreten, sind vier R-C-Kombinationen (116, 117; 118, 119; 120, 121; 122, 123) sowie die Drossel 84 zur Entstörung vorgesehen.

Im automatischen Regelbetrieb des erfindungsgemäßen Bestrahlungsgerätes ist die Wärmeabgabe der Heizung 79 des Fönes nicht von Bedeutung; d.h. die mit der Regelung einhergehende unterschiedliche Wärmeabgabe der Heizung 79 macht sich nicht störend bei der Aufrechterhaltung eines ganz bestimmten voreingestellten Betriebszustandes bemerkbar. Mit Hilfe der in Fig. 7 dargestellten Regelschaltung läßt sich die Temperatur der UV-Lampe 33 konstant halten bzw. auf einen beliebigen Wert einstellen, der dann konstant beibehalten wird. Da sowohl die Strahlungsleistung als auch die abgegebene Wellenlänge der Strahlung der UV-Lampe 33 von ihrer Betriebstemperatur abhängig ist, läßt sich durch Regelung der Betriebstemperatur die gewünschte Strahlungsleistung und auch Wellenlänge einstellen. Mit Hilfe des erfindungsgemäßen Behandlungsgerätes läßt sich also der für jeden Behandlungszweck optimale Arbeitspunkt der UV-Lampe 33 einstellen. Darüber hinaus ermöglicht die geregelte Kühlung, daß das erfindungsgemäße Bestrahlungsgerät im Langzeitbetrieb eingesetzt werden kann.

Nach einem weiteren Ausführungsbeispiel der Erfindung ist vorgesehen, daß an dem Gehäuse 1 des UV-Aufsatzes 143 ein Abstandshalter 128 befestigt ist (Fig. 8 ). Der Abstandshalter 128 besteht aus einem ebenen Gitter 129 aus Kunststoff oder Draht, das etwa die Größe der dem Bestrahlungsobjekt zugewandten Seite des UV-Aufsatzes 143 besitzt. An dem Gitter 129 sind einendig Abstandstreben 130 befestigt, die rechtwinklig zu der Gitterebene verlaufen und anderendig in Richtung der Längsachse 11 verschieblich sowie arretierbar an dem Gehäuse 1 geführt sind. Die Randbereiche 131 des Gitters 129 sind in Richtung auf das Gehäuse 1 abgewinkelt. Durch die Längsverschieblichkeit kann das Gitter auf einen vorgegebenen Abstand zur UV-Lampe 33 des UV-Aufsatzes 143 eingestellt werden. Zur genauen Ermittlung des Abstands kann ggf. eine an einer Abstandstrebe 130 angeordnete Stricheinteilung 132 zu Hilfe genommen werden. Ein an dem Gehäuse 1 drehbar gelagertes Zahnrad 133 kämmt mit einer Zahnung 134 einer der Abstandstreben 130. Das Zahnrad ist einstückig sowie koaxial mit einem Bedienknopf 135 verbunden, der außerhalb des Gehäuses 1 angeordnet ist. Durch manuelle Drehung des Bedienknopfes 135 läßt sich somit der Abstandshalter 128 in Richtung des in Fig. 9 eingetragenen Doppelpfeils 136 verfahren. Mit Hilfe des Abstandshalters 128 kann der Anwender des erfindungsgemäßen Bestrahlungsgerätes die Entfernung zwischen Behandlungsstelle (z. B. Haut) und UV-Lampe 33 genau einhalten. Hierzu wird das Gitter 129 gegen die zu bestrahlende Behandlungsstelle gedrückt. Gerade an schlecht zugänglichen Behandlungsstellen, z. B. Behandlung der Kopfhaut im Hinterkopfbereich, erweist sich der Abstandhalter 128 als besonders hilfreich, da er durch Wahrung des genauen Bestrahlungsabstandes eine optimale Behandlung ermöglicht.

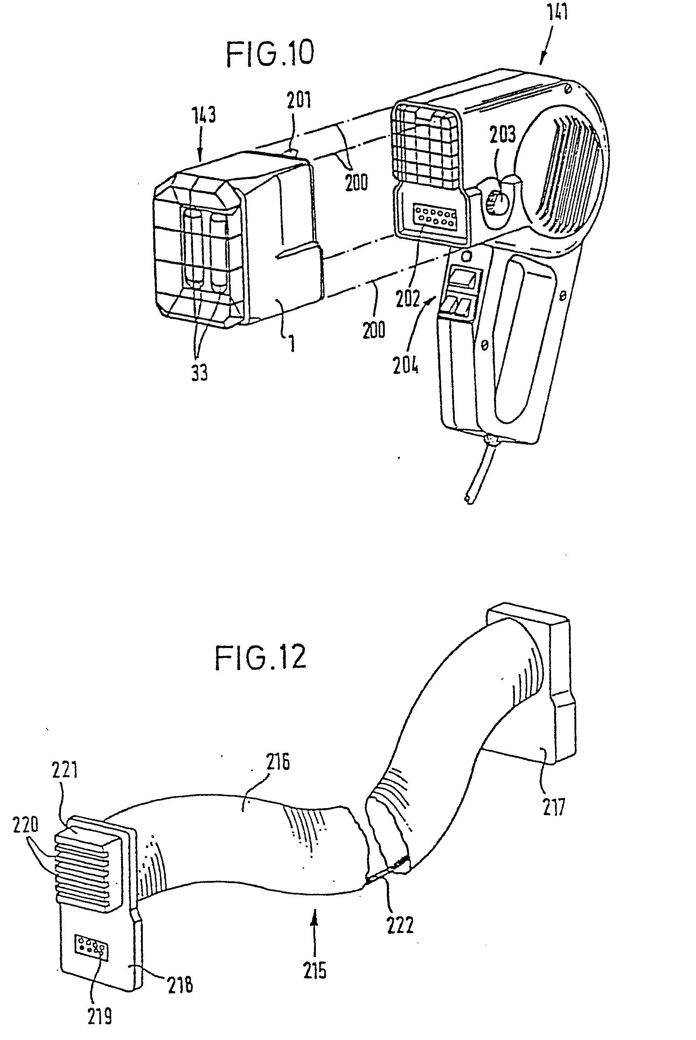

In der Fig. 10 ist ein weiteres Ausführungsbeispiel des erfindungsgemäßen Bestrahlungsgerätes gezeigt. Hierbei sind Haarfön 141 sowie UV-Aufsatz 143 voneinander getrennt. Der UV-Aufsatz 143 kann entlang der gestrichelt gezeichneten Geraden 200 in Richtung auf den Haarfön 141 bewegt werden, bis beide Teile mittels einer Rastfeder 201, die sich, in der Fig. 10 gesehen, im oberen Bereich des UV-Aufsatzes 143 befindet, verrasten. In der verrasteten Stellung werden die notwendigen elektrischen Verbindungen zwischen dem Haarfön 141 und dem UV-Aufsatz 143 mittels einer am Haarfön 141 angeordneten Buchse 202 und einem entsprechenden, nicht dargestellten Steckerteil an dem UV-Aufsatz 143 hergestellt. Ein seitlich an dem Haarfön 141 angebrachter Drehknopf 203 dient zum Einstellen der Bestrahlungszeit; ist also mit dem Potentiometer 104 der in der Fig. 7 dargestellten Zeitschaltung 7 verbunden. Die insgesamt mit 204 bezeichneten Bedienelemente stellen einen Schalter für das Gebläse des Fönes 141, Schalter für die im Gehäuse 1 des UV-Aufsatzes 143 angeordneten UV-Lampen 33 sowie einen Taster zum Starten der Zeitschaltung 71 (Fig. 7) dar.

Hierbei ist das Fotoelement mit dem zugehörigen Filter erfindungsgemäß im Aufsatz angeordnet.

In der Fig. 11 ist der Fön 141 der Fig. 10 in geöffnetem Zustand dargestellt. In das Föngehäuse 205 ist ein geschlossener, bügelförmiger Handgriff 206 integriert. Dieser Handgriff weist, in der Fig. 11 betrachtet, auf der linken oberen Seite die Bedienelemente 204 auf. Darunter sind Bauelemente 207 angeordnet, die der Entstörung der erfindungsgemäßen, in Fig. 7 dargestellten Schaltung dienen. In die rechte Seite des Handgriffs 206 ist eine mit Bauelementen bestückte Platine 208 eingesetzt, die in ihrem unteren Bereich die Zeitschaltung 71, daran nach oben anschließend das Relais 103 und im oberen Bereich die Phasenanschnittschaltung 73 aufweist. Im Kopfbereich des Fönes 141 ist das Gebläse 140 angeordnet, das halbkreisförmig von den Kondensatoren 95, 96 und 97 umgeben wird. Diese Kondensatoren gehören zu dem Vorwiderstand 94 der UV-Lampenschaltung 69. Die weiteren drei an die vorgenannten Kondensatoren anschliessenden Kondensatoren 95', 96' und 97' gehören zu dem Vorwiderstand der zweiten UV-Lampenschaltung 70. Weitere, zu den Vorwiderständen gehörende Bauelemente (Widerstände) sind durch die Bezugsziffern 209 sowie 209' angedeutet. Unterhalb des Gebläses 140 sind auf der Platine 210 die Leistungswiderstände 98 bzw. 98' der Vorwiderstände sowie weitere Entstörglieder der erfindungsgemäßen Schaltung angeordnet.

Der Luftaustrittstutzen 142 des Fönes 141 ist mittels eines Gitters 211 abgedeckt, so daß ein unbeabsichtigtes Berühren der dort angeordneten Heizung 79 verhindert wird. Die Heizung besteht aus in sich gewendeltem Widerstandsdraht 212, der auf einen temperaturbeständigen Wickelkörper 213 aufgewickelt ist. An dem Wickelkörper 213 ist der schon aus der Fig. 7 bekannte Temperaturschalter 80 angeordnet.

Unterhalb des Luftaustrittstutzens 142 ist die Buchse 202 befestigt; des weiteren befinden sich hier die Bauteile 214, die der Motorsiebung dienen.

Nach einem weiteren Ausführungsbeispiel der Erfindung ist vorgesehen, den UV-Aufsatz mittels einer in Fig. 12 dargestellten flexiblen Verbindungsvorrichtung 215 an dem Fön zu befestigen. Die Verbindungsvorrichtung 215 besteht aus einem flexiblen Schlauch 216, der an seinem einen Ende mit einer Tülle 217 verbunden ist, die auf den Luftaustrittstutzen des Fönes (nicht dargestellt) aufsteckbar ist. An seinem anderen Ende ist eine Platte 218 befestigt, die, in der Fig. 12 betrachtet, in ihrem unteren Bereich eine Buchse 219 für den UV-Aufsatz 143 aufweist, der auf einen mit Luftaustrittsschlitzen 220 versehenen Ansatz 221, der mit dem Schlauch 216 in Verbindung steht, aufsetzbar ist. Die Buchse 219 ist über ein in dem flexiblen Schlauch 216 verlaufenden Versorgungskabel 222 und weiteren nicht dargestellten Verbindungselementen mit dem nicht dargestellten Fön verbunden. Diese Verbindungsvorrichtung 215 ermöglicht es, den Fön 141 stationär in einem Halter od.dgl. anzuordnen und mittels des auf die Verbindungsvorrichtung 215 aufgesetzten UV-Aufsatzes 143 die Bestrahlungsbehandlung vorzunehmen. Der Anwender braucht hierbei nur den relativ leichten, an der Verbindungsvorrichtung 215 angeschlossenen UV-Aufsatz 143 halten; darüber hinaus ermöglicht der flexible Schlauch 116 auch eine Behandlung an schlecht zugänglichen Körperstellen.

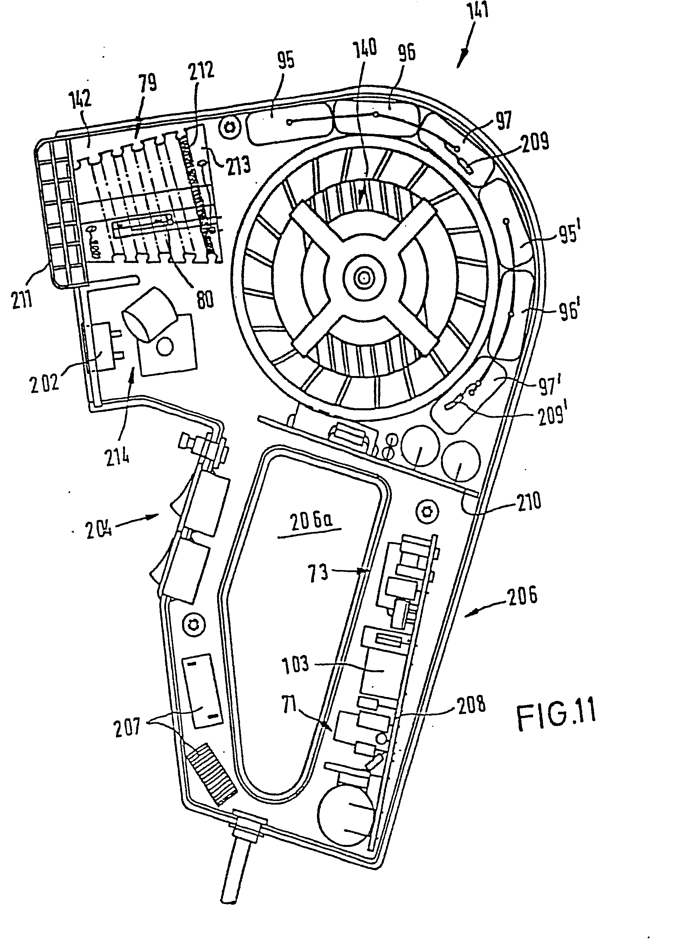

In der Fig. 13 ist ein weiteres Ausführungsbeispiel eines erfindungsgemäßen UV-Aufsatzes 143 von der Bestrahlungsseite her gezeigt. Die Front dieses UV-Aufsatzes 143 weist zwei nebeneinander angeordnete Reflektoren 34 auf, in deren Mitte sich jeweils eine UV-Lampe 33 befindet. Unterhalb der UV-Lampen 33 sind Luftaustrittsöffnungen 223 angeordnet, durch die ein Teil des von dem Fön erzeugten Luftstromes strömt und somit die UV-Lampen 33 kühlt. Die Luftaustrittsöffnungen 223 erstrecken sich jeweils über die gesamte Länge der UV-Lampen 33 und sind auf einer geraden Linie mit gleichmäßigem Abstand voneinander angeordnet. Beidseitig der Front des UV-Aufsatzes 143 befindet sich jeweils ein parallel zu den UV-Lampen 33 verlaufender Luftaustrittsschacht 224. Bei dem Betrieb des erfindungsgemäßen Strahlungsgerätes strömt aus diesen beiden Luftaustrittsschächten 224 ein Teil des von dem Fön erzeugten Luftstromes aus, der dann von dem Anwender auf die Behandlungsstelle geleitet wird.

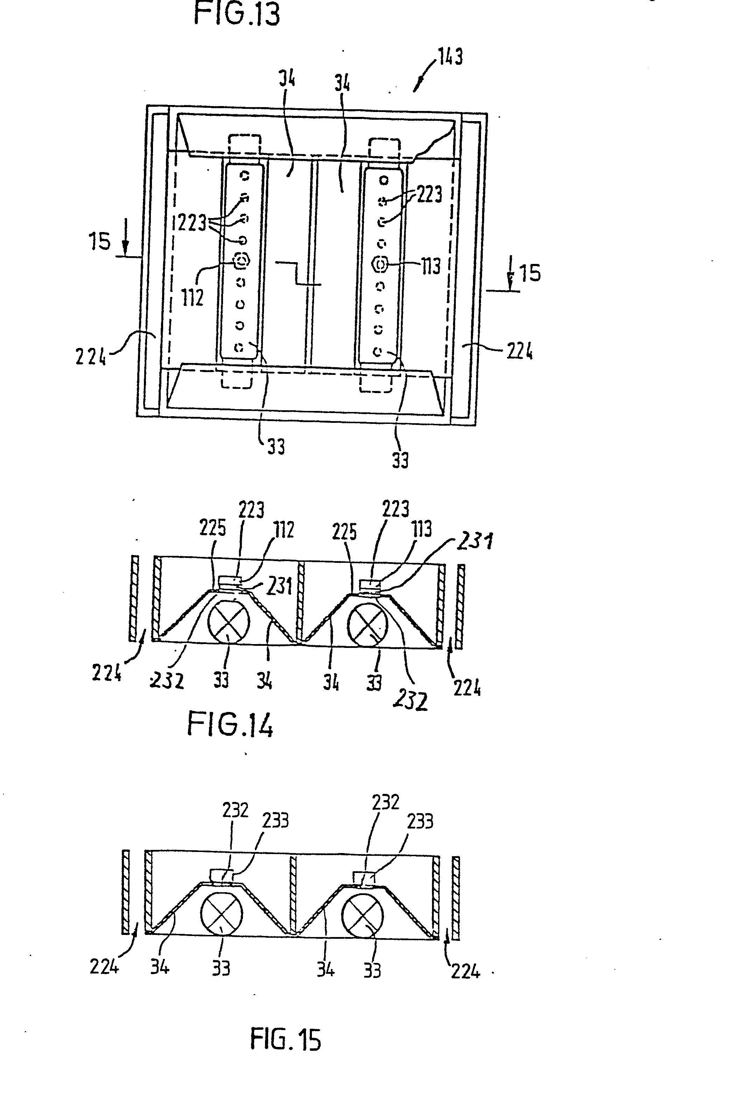

In der Fig. 14 ist ein Schnitt durch den Frontbereich des UV-Aufsatzes 143 gemäß der Fig. 13 entlang der Linie 15-15 gezeigt. Nochmals sind hier die Luftaustrittsöffnungen 223 eingezeichnet, die mit der jeweiligen UV-Lampe 33 fluchten. Auf der der jeweiligen UV-Lampe 33 abgewandten Seite 225 des jeweiligen Reflektors 34 sind hinter der Öffnung 232 die beiden aus der Fig. 7 bekannten Foto-Widerstände 112 bzw. 113 angeordnet. Diese Foto-Widerstände dienen der Ermittlung der UV-Lampen-Strahlungsleistung, wobei diesen die nur UV-Licht durchlassenden Filter 231 vorgeordnet sind.

In Fig. 15 ist ein Schnitt durch den Frontbereich des UV-Aufsatzes 143 gezeigt in einer weiteren Ausführungsform der Erfindung. Der Reflektor 34 hinter den Lampen 8 weist zusätzlich zu den Luftaustrittsöffnungen im Bereich der Lampen 33 eine Strahlungsöffnung 232 auf, vor der in Strömungsrichtung eine Glimmlampe 233 angeordnet ist. Diese erfindungsgemäße Glimmlampe dient zur Ermittlung und zur Regelung der UV-Lampentemperatur.

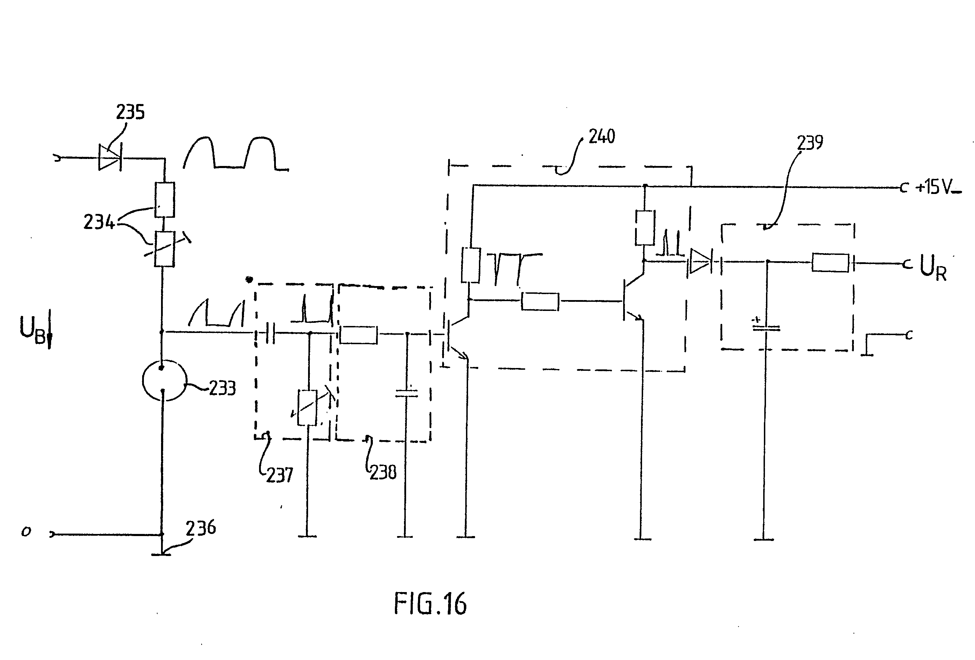

Die erfindungsgemäße Glimmlampe wird mit folgender in Fig. 16 dargestellter Meßschaltung betrieben. Die Glimmlampe 233 mit niedrigem Durchlaßstrom wird über einen entsprechenden Vorwiderstand 234 an eine hohe pulsierende Gleichspannungsquelle $U_B$ über eine Gleichrichterdiode 235 gelegt. Die Spannung an der Glimmlampe 233 steigt bei jedem Puls der Gleichspannungsquelle bis zur Durchbruchsspannung an. In diesem Moment wird dann die Glimmlampe niederohmig und leitet die Spannung auf den Fußpunkt 236 der Schaltung ab. Über einen nachgeschalteten Hochpaß 237 wird eine der Durchbruchspannung entsprechende Spannungsspitze abgenommen, die anschließend einem Tiefpaß 238 zur Entstörung zugeleitet wird. Die derart entstörten Spannungsspitzen werden in eine entsprechende Gleichspannung aufintegriert, und zwar mittels eines Integrators 239. Dabei erfolgt zweckmäßigerweise eine Zwischenverstärkung der Spannungsimpulse zwischen dem Ausgang des Tiefpasses 238 und dem Integrator 239 mittels einer üblichen Verstärkerschaltung 240. Die Ausgangsspannung $U_R$ des Integrators 239 kann nun erfindungsgemäß als Regelspannung verwendet werden. Dabei kann mittels dieser Regelspannung entweder die Betriebsspannung der UV-Lampe geregelt werden oder aber die Leistung des Gebläses des Haartrockners 141, so daß dann entsprechend dem Anstieg bzw. dem Abfall der Regelspannung entweder die Betriebsspannung des UV-Strahlers oder die Betriebsspannung des Gebläses erhöht oder erniedrigt wird. Dabei erfolgt die Regelung derart, daß bei einer Erniedrigung der Regelspannung, was einer verstärkten Ionisierung des Gases der Glimmlampe und damit einer Erniedrigung der Durchbruchspannung entspricht, eine Herabsetzung der Betriebsspannung des UV-Strahlers bzw. eine Erhöhung der Kühlleistung des Gebläses erfolgt.

Die Erfindung weist folgende Vorteile zusammenfassend auf:

Es kann eine Strahlungsenergiemessung durchgeführt werden, wobei die Energiedichte der Wellenlänge berücksichtigt wird. Filtervorsätze sind nicht erforderlich, so daß sich insgesamt eine äußerst billige Lösung ergibt, wobei eine gute Temperaturunempfindlichkeit besteht. Die erfindungsgemäße Meßschaltung ist geringen Alterserscheinungen unterworfen und kann mittels sehr einfacher Schaltungstechnik realisiert werden.

Die in den Fig. 10,11,13,14 und 15 dargestellten Ausführungsbeispiele des erfindungsgemäßen Bestrahlungsgerätes weisen jeweils zwei UV-Lampen 33 auf. Hierbei ist es zweckmäßig, unterschiedliche UV-Lampen zu verwenden; so kann z.B. einmal eine UV- A -Strahlung und zum anderen eine UV-B-A-Strahlung abgebende Lampe verwendet werden. Nach einem anderen Ausführungsbeispiel ist es jedoch auch denkbar, daß die in den Fig. 10, 11, 13 und 14 dargestellten Gegenstände jeweils nur eine UV-Lampe aufweisen.

In den dargestellten Ausführungsbeispielen ist der UV-Strahler jeweils in einem separaten, auf den Luftaustrittsstutzen aufsetzbaren Teil angeordnet. Die Erfindung bezieht sich jedoch auch auf die Anordnung des UV-Strahlers unmittelbar im Föngehäuse, und zwar im Bereich des Luftaustrittsstutzens, wobei im übrigen die elektrische Schaltung zur Betreibung des erfindungsgemäßen Bestrahlungsgerätes und die Ausbildung des Strahlers und die Führung des Luftstromes, wie im vorstehenden erläutert, ausgeführt sein können.

Die Erfindung umfaßt auch alle im Sinne der Erfindung gleichwirkenden Merkmale und Ausführungsformen.

II/mj/M 366

---

Ansprüche:

1. Bestrahlungsgerät, kombiniert aus einem Gebläse und einer Strahlungsquelle, die ultraviolette Strahlung (UV-Strahlung) abgibt und über einen Vorwiderstand an die Netzspannung einer Wechselspannungsquelle angeschlossen ist, wobei die Strahlungsquelle mit dem vom Gebläse erzeugten Luftstrom zusammenwirkt und wobei die Drehzahl des Gebläses in Abhängigkeit von der Erhitzung der UV-Lampe geregelt wird, d a d u r c h g e k e n n z e i c h n e t, daß die Regelung der Drehzahl des Gebläses (140) in Abhängigkeit von der emittierten UV-Strahlung der UV-Lampe erfolgt.

2. Bestrahlungsgerät nach Anpruch 1, d a d u r c h g e k e n n z e i c h n e t, daß die UV-Strahlung mittels eines Fotoelementes (230) erfaßt wird, das von einem ausschließlich für UV-Strahlung durchlässigen Filter (231) abgedeckt ist.

3. Bestrahlungsgerät nach Anspruch 1, d a d u r c h g e k e n n z e i c h n e t, daß die UV-Strahlung mittels einer Gasentladungslampe (233) erfaßt wird, und die Durchbruchspannung der Gasentladungslampe als Regelgröße für die Regelung der Strahlertemperatur des UV-Strahlers verwendet wird.

4. Bestrahlungsgerät nach Anspruch 3, d a d u r c h g e k e n n z e i c h n e t, daß mittels eines vor der Gasentladungslampe (233) im Strahlengang des UV-Strahlers (33) angeordneten Filters eine Frequenzselektion erfolgt.

5. Bestrahlungsgerät nach Anspruch 1, d a d u r c h g e k e n n z e i c h n e t, daß als Gasentladungslampe eine Glimmlampe verwendet wird.

6. Bestrahlungsgerät nach Anspruch 2, d a d u r c h g e k e n n z e i c h n e t, daß das Fotoelement (230) als Fotowiderstand ausgebildet ist.

7. Bestrahlungsgerät nach Anspruch 2, d a d u r c h g e k e n n z e i c h n e t, daß der Filter (231) mit dem Fotoelement (230) hinter einer Öffnung (232) im Reflektor der UV-Lampe (33) angeordnet ist.

8. Bestrahlungsgerät nach Anspruch 3, d a d u r c h g e k e n n z e i c h n e t, daß die Gasentladungslampe über einen Vorwiderstand an eine hochpulsierende Gleichspannung gelegt ist und an der Gasentladungslampe ein Hochpaß angeschlossen ist, dessen Ausgangsspannungsimpulse auf einen Integrator geleitet werden, dessen Ausgangsgleichspannung die Regelgröße bildet.

9. Bestrahlungsgerät nach einem oder mehreren der Ansprüche 1 bis 8, dadurch gekennzeichnet, daß in Reihe zu einer als Vorwiderstand dienenden Heizwicklung (79) an deren Abgriff der als Gleichstrommotor (83) ausgebildete Motor über eine Diode (82) angeschlossen ist, ein Tyristor (109) einer Phasenanschnittsschaltung (73) zum Regeln der Motordrehzahl des Motors (83) geschaltet ist und das phasenanschnittswinkelbestimmende Glied (107) der Phasenanschnittsschaltung (73) mindestens ein vorzugsweise als Fotowiderstand ausgebildetes Fotoelement (230; 112, 113) oder die Gasentladungslampe (233) ist.

10. Bestrahlungsgerät nach Anspruch 9, dadurch gekennzeichnet, daß zu dem Fotowiderstand (112, 113) ein Motordrehzahleinstelltrimmer (114) zur Arbeitspunkteinstellung der UV-Lampe in Reihe geschaltet ist.

11. Bestrahlungsgerät nach Anspruch 9, dadurch gekennzeichnet, daß die Motordrehzahl des Gebläses durch die Phasenanschnittsschaltung (73) derart geregelt wird, daß die UV-Lampe eine konstante, vorwählbare Temperatur aufweist.

FIG.1

FIG. 2
34
232
39
33
39
37
38
36

FIG.3
s(λ) in (%)
Nr. 1: EDELSTAHL GOLD-BELEGT (GLÄNZEND)
Nr. 2: ALUMINIUM - MATT (SANDSTRAHL)
Nr. 3: ALUMINIUM GELB ELOXIERT
Nr. 4: ALUMINIUM BLAU ELOXIERT
Nr. 5: ALUMINIUM PURPUR ELOXIERT
Nr. 6: EDELSTAHL GLÄNZEND (CHROM)
70
60
50
40
30
20
10
250
300
350
λ in nm
1
2
3
4
5
6

FIG.4
41
45
46
33
42
10
9
10a
47
49
48
19
17
51
49
50
220V/50 Hz

FIG.5
51
52
1A
53
100Ω
100n
56
4x
1N 4001
54
55
57
58
1N 4001
LM317
270Ω
61
5kΩ
7809
7806
1N 4001
59
3-20V
2A
60
0V
+9V 1A
+6V 1A

+U DD 15V
(8-18V)
62
24 x LED GL 9 P 4 od CQV
63
1k
65
RTϑ
66
ZD
5,6V
2x
100k
100k
18 17 16 15 14 13 12 11 10
UAA 180
1 2 3 4 5 6 7 8 9
64
U Motor
67
68
10k
2x
100k
ZP
5,6V
18 17 16 15 14 13 12 11 10
UAA 180
1 2 3 4 5 6 7 8 9

FIG.6

FIG.7

0185139

11
129
131
132
131
128
136
135
130
130
130
133
1
134
143
142
141
140
131
129
131
131
128
131

FIG.8

FIG.9

0185139

FIG.10
141
143
201
200
203
202
200
1
204
33

FIG.12
216
221
220
217
222
215
218
219

141
140
95
96
212
79
142
213
97
209
95'
96'
97'
209'
211
80
202
214
210
204
206a
73
206
103
71
208
207

FIG.11

0185139

FIG.13

143
34
34
223
223
15
15
112
113
224
224
33
33

223
225
112
223
225
113
231
231
224
232
33
34
34
33
232
224

FIG.14

232
233
232
233
224
34
33
34
33
224

FIG. 15

$U_B$
233
234
235
236
237
238
239
240
$U_R$
+15V

FIG.16

Europäisches Patentamt

# EUROPÄISCHER RECHERCHENBERICHT

Nummer der Anmeldung

EP 85110623.7

| EINSCHLÄGIGE DOKUMENTE | | | KLASSIFIKATION DER ANMELDUNG (Int Cl 4) |
|---|---|---|---|
| Kategorie | Kennzeichnung des Dokuments mit Angabe, soweit erforderlich, der maßgeblichen Teile | Betrifft Anspruch | |
| D,Y | DE - A1 - 3 322 071 (KERSCHGENS) | 1,9 | A 61 N 5/06 |
| A | * Gesamt * | 8,10, 11 | H 02 P 7/00 |
| | & EP-A1-0 104 466 | | G 05 F 1/10 |
| | -- | | G 01 J 1/42 |
| Y | DE - A1 - 3 016 773 (PAUL B. ELDER) | 1,9 | |
| A | * Ansprüche 1,10; Seite 15, Zeilen 3-5; Fig. 1,2 * | 2,6,7 | |
| | -- | | |
| P,Y | DE - A1 - 3 404 214 (KERSCHGENS) | 1,9 | |
| | * Ansprüche 1,7-10; Fig. 1,2 * | | |
| | & EP-A1-0 158 025 | | |
| | -- | | |
| Y | DE - A1 - 2 943 674 (PAUL B. ELDER) | 1,9 | |
| A | * Ansprüche 1,3; Seite 12, Zeilen 9-19; Fig. 1,2 * | 2,6,7 | RECHERCHIERTE SACHGEBIETE (Int Cl 4) |
| | -- | | |
| A | DE - A1 - 3 232 963 (BARTELS) | 1,3,5, 8 | A 61 N |
| | * Seite 6, Zeile 34 - Seite 7, Zeile 2; Fig. 1 * | | H 02 P |
| | -- | | G 05 F |
| A | DE - A1 - 3 042 084 (AUTARK) | 1,2,6 | G 01 J |
| | * Anspruch 2; Fig. * | | |
| | -- | | |
| A | DE - A1 - 2 531 381 (BIVIATOR) | 1,2,6 | |
| | * Ansprüche 1,3-5; Fig. 1,5 * | | |
| | -- | | |
| A | US - A - 3 971 943 (JEUNEHOMME) | 1 | |
| | * Zusammenfassung; Fig. 2,3 * | | |
| | -- | | |

Der vorliegende Recherchenbericht wurde für alle Patentansprüche erstellt

| Recherchenort | Abschlußdatum der Recherche | Prüfer |
|---|---|---|
| WIEN | 22-01-1986 | NEGWER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches
Patentamt

# EUROPÄISCHER RECHERCHENBERICHT

Nummer der Anmeldung

-2-

EP 85110623.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | US - A - 4 279 254 (BOSCHETTI)<br>* Spalte 2, Zeilen 43-49; Fig. 1 *<br>---- | 1 | |
| | | | RECHERCHIERTE SACHGEBIETE (Int Cl 4) |
| | | | |

Der vorliegende Recherchenbericht wurde für alle Patentansprüche erstellt

| Recherchenort | Abschlußdatum der Recherche | Prüfer |
|---|---|---|
| WIEN | 22-01-1986 | NEGWER |

KATEGORIE DER GENANNTEN DOKUMENTEN

X von besonderer Bedeutung allein betrachtet
Y von besonderer Bedeutung in Verbindung mit einer anderen Veröffentlichung derselben Kategorie
A technologischer Hintergrund
O nichtschriftliche Offenbarung
P Zwischenliteratur
T der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82